# EUROPEAN PATENT SPECIFICATION

(11) **EP 2 462 945 B1**
(45) Date of publication and mention of the grant of the patent: **04.06.2014**
(21) Application number: 12157644.1
(22) Date of filing: 16.08.2007
(51) Int. Cl.: A61K 38/57, A61K 38/30, A61P 35/00, C07K 14/65, C07K 14/81, C12N 9/64

(54) **METHOD OF INHIBITING ANGIOGENESIS, TUMORIGENESIS AND CATHEPSIN ACTIVITY**
HEMMUNG DER ANGIOGENESE, TUMORIGENESE UND CATHEPSIN-AKTIVITÄT MIT INSULINARTIGEN WACHSTUMSFAKTOR BINDENDEM PROTEIN
PROCÉDÉ D'INHIBITION DE L'ANGIOGENÈSE, DE LA TUMORIGENÈSE ET DE L'ACTIVITÉ DE LA CATHEPSINE À L'AIDE D'UNE PROTÉINE DE LIAISON AU FACTEUR DE CROISSANCE DE TYPE INSULINE

(30) Priority: 16.08.2006 US 837930 P
(43) Date of publication of application: 13.06.2012
(62) Divisional of application: 07800446.2
(73) Proprietor: National Research Council of Canada, Ottawa, ON K1A 0R6 (CA)
(72) Inventor: Moreno, Maria J., Ottawa, Ontario K1A 0R6 (CA)
(74) Representative: Jostarndt, Hans-Dieter

(56) References cited:
- WO-A2-00/23469
- WO-A2-2004/080379
- WO-A2-2006/034832

### SEQUENCE LISTING

<110> National Research Council of Canada
<120> METHOD OF INHIBITING ANGIOGENESIS, TUMORIGENESIS AND CATHEPSIN ACTIVITY
<130> 11617-PT-WO-EP-TLG1
<140> 07800446.2
   <141> 2007-08-16
<150> US 60/837,930
   <151> 2006-08-16
<160> 25
<170> PatentIn version 3.3
<210> 1
   <211> 272
   <212> PRT
   <213> human
<400> 1
   Met Ser Glu Val Pro Val Ala Arg Val Trp Leu Val Leu Leu Leu Leu
   1               5                   10                  15
   Thr Val Gln Val Gly Val Thr Ala Gly Ala Pro Trp Gln Cys Ala Pro
               20                  25                  30
   Cys Ser Ala Glu Lys Leu Ala Leu Cys Pro Pro Val Ser Ala Ser Cys
           35                  40                  45
   Ser Glu Val Thr Arg Ser Ala Gly Cys Gly Cys Cys Pro Met Cys Ala
       50                  55                  60
   Leu Pro Leu Gly Ala Ala Cys Gly Val Ala Thr Ala Arg Cys Ala Arg
   65                  70                  75                  80
   Gly Leu Ser Cys Arg Ala Leu Pro Gly Glu Gln Gln Pro Leu His Ala
                   85                  90                  95
   Leu Thr Arg Gly Gln Gly Ala Cys Val Gln Glu Ser Asp Ala Ser Ala
               100                 105                 110
   Pro His Ala Ala Glu Ala Gly Ser Pro Glu Ser Pro Glu Ser Thr Glu
           115                 120                 125
   Ile Thr Glu Glu Glu Leu Leu Asp Asn Phe His Leu Met Ala Pro Ser
       130                 135                 140
   Glu Glu Asp His Ser Ile Leu Trp Asp Ala Ile Ser Thr Tyr Asp Gly
   145                 150                 155                 160
   Ser Lys Ala Leu His Val Thr Asn Ile Lys Lys Trp Lys Glu Pro Cys
                   165                 170                 175
   Arg Ile Glu Leu Tyr Arg Val Val Glu Ser Leu Ala Lys Ala Gln Glu
               180                 185                 190
   Thr Ser Gly Glu Glu Ile Ser Lys Phe Tyr Leu Pro Asn Cys Asn Lys
           195                 200                 205
   Asn Gly Phe Tyr His Ser Arg Gln Cys Glu Thr Ser Met Asp Gly Glu
       210                 215                 220
   Ala Gly Leu Cys Trp Cys Val Tyr Pro Trp Asn Gly Lys Arg Ile Pro
   225                 230                 235                 240
   Gly Ser Pro Glu Ile Arg Gly Asp Pro Asn Cys Gln Ile Tyr Phe Asn
                   245                 250                 255
   Val Gln Asn Thr Gly His His His His His His His His Gly Gly Gln
               260                 265                 270
<210> 2
   <211> 128
   <212> PRT
   <213> artificial
<220>
   <223> C-terminal fragment of IGFBP-1
<400> 2
<210> 3
   <211> 352
   <212> PRT
   <213> human
<400> 3
   Met Leu Pro Arg Val Gly Cys Pro Ala Leu Pro Leu Pro Pro Pro Pro
   1               5                   10                  15
   Leu Leu Pro Leu Leu Pro Leu Leu Leu Leu Leu Leu Gly Ala Ser Gly
               20                  25                  30
   Gly Gly Gly Gly Ala Arg Ala Glu Val Leu Phe Arg Cys Pro Pro Cys
           35                  40                  45
   Thr Pro Glu Arg Leu Ala Ala Cys Gly Pro Pro Pro Val Ala Pro Pro
       50                  55                  60
   Ala Ala Val Ala Ala Val Ala Gly Gly Ala Arg Met Pro Cys Ala Glu
   65                  70                  75                  80
   Leu Val Arg Glu Pro Gly Cys Gly Cys Cys Ser Val Cys Ala Arg Leu
                   85                  90                  95
   Glu Gly Glu Ala Cys Gly Val Tyr Thr Pro Arg Cys Gly Gln Gly Leu
               100                 105                 110
   Arg Cys Tyr Pro His Pro Gly Ser Glu Leu Pro Leu Gln Ala Leu Val
           115                 120                 125
   Met Gly Glu Gly Thr Cys Glu Lys Arg Arg Asp Ala Glu Tyr Gly Ala
       130                 135                 140
   Ser Pro Glu Gln Val Ala Asp Asn Gly Asp Asp His Ser Glu Gly Gly
   145                 150                 155                 160
   Leu Val Glu Asn His Val Asp Ser Thr Met Asn Met Leu Gly Gly Gly
                   165                 170                 175
   Gly Ser Ala Gly Arg Lys Pro Leu Lys Ser Gly Met Lys Glu Leu Ala
               180                 185                 190
   Val Phe Arg Glu Lys Val Thr Glu Gln His Arg Gln Met Gly Lys Gly
           195                 200                 205
   Gly Lys His His Leu Gly Leu Glu Glu Pro Lys Lys Leu Arg Pro Pro
       210                 215                 220
   Pro Ala Arg Thr Pro Cys Gln Gln Glu Leu Asp Gln Val Leu Glu Arg
   225                 230                 235                 240
   Ile Ser Thr Met Arg Leu Pro Asp Glu Arg Gly Pro Leu Glu His Leu
                   245                 250                 255
   Tyr Ser Leu His Ile Pro Asn Cys Asp Lys His Gly Leu Tyr Asn Leu
               260                 265                 270
   Lys Gln Cys Lys Met Ser Leu Asn Gly Gln Arg Gly Glu Cys Trp Cys
           275                 280                 285
   Val Asn Pro Asn Thr Gly Lys Leu Ile Gln Gly Ala Pro Thr Ile Arg
       290                 295                 300
   Gly Asp Pro Glu Cys His Leu Phe Tyr Asn Glu Gln Gln Glu Ala Arg
   305                 310                 315                 320
   Gly Val His Thr Gln Arg Met Gln Gly Ser Gly Trp Ser His Pro Gln
                   325                 330                 335
   Phe Glu Lys Thr Gly His His His His His His His His Gly Gly Gln
               340                 345                 350
<210> 4
   <211> 165
   <212> PRT
   <213> artificial
<220>
   <223> C-terminal fragment of IGFBP-2
<400> 4
   Met Leu Leu Leu Leu Leu Leu Leu Gly Leu Arg Leu Gln Leu Ser Leu
   1               5                   10                  15
   Gly Ile Ala Ser Lys Gly Gly Lys His His Leu Gly Leu Glu Glu Pro
               20                  25                  30
   Lys Lys Leu Arg Pro Pro Pro Ala Arg Thr Pro Cys Gln Gln Glu Leu
           35                  40                  45
   Asp Gln Val Leu Glu Arg Ile Ser Thr Met Arg Leu Pro Asp Glu Arg
       50                  55                  60
   Gly Pro Leu Glu His Leu Tyr Ser Leu His Ile Pro Asn Cys Asp Lys
   65                  70                  75                  80
   His Gly Leu Tyr Asn Leu Lys Gln Cys Lys Met Ser Leu Asn Gly Gln
                   85                  90                  95
   Arg Gly Glu Cys Trp Cys Val Asn Pro Asn Thr Gly Lys Leu Ile Gln
               100                 105                 110
   Gly Ala Pro Thr Ile Arg Gly Asp Pro Glu Cys His Leu Phe Tyr Asn
           115                 120                 125
   Glu Gln Gln Glu Ala Arg Gly Val His Thr Gln Arg Met Gln Asp Pro
       130                 135                 140
   Trp Ser His Pro Gln Phe Glu Lys Thr Gly His His His His His His
   145                 150                 155                 160
   His His Gly Gly Gln
                   165
<210> 5
   <211> 282
   <212> PRT
   <213> human
<400> 5
   Met Leu Pro Leu Cys Leu Val Ala Ala Leu Leu Leu Ala Ala Gly Pro
   1               5                   10                  15
   Gly Pro Ser Leu Gly Asp Glu Ala Ile His Cys Pro Pro Cys Ser Glu
               20                  25                  30
   Glu Lys Leu Ala Arg Cys Arg Pro Pro Val Gly Cys Glu Glu Leu Val
           35                  40                  45
   Arg Glu Pro Gly Cys Gly Cys Cys Ala Thr Cys Ala Leu Gly Leu Gly
       50                  55                  60
   Met Pro Cys Gly Val Tyr Thr Pro Arg Cys Gly Ser Gly Leu Arg Cys
   65                  70                  75                  80
   Tyr Pro Pro Arg Gly Val Glu Lys Pro Leu His Thr Leu Met His Gly
                   85                  90                  95
   Gln Gly Val Cys Met Glu Leu Ala Glu Ile Glu Ala Ile Gln Glu Ser
               100                 105                 110
   Leu Gln Pro Ser Asp Lys Asp Glu Gly Asp His Pro Asn Asn Ser Phe
           115                 120                 125
   Ser Pro Cys Ser Ala His Asp Arg Arg Cys Leu Gln Lys His Phe Ala
   130 135 140
   Lys Ile Arg Asp Arg Ser Thr Ser Gly Gly Lys Met Lys Val Asn Gly
   145 150 155 160
   Ala Pro Arg Glu Asp Ala Arg Pro Val Pro Gln Gly Ser Cys Gln Ser
   165 170 175
   Glu Leu His Arg Ala Leu Glu Arg Leu Ala Ala Ser Gln Ser Arg Thr
   180 185 190
   His Glu Asp Leu Tyr Ile Ile Pro Ile Pro Asn Cys Asp Arg Asn Gly
   195 200 205
   Asn Phe His Pro Lys Gln Cys His Pro Ala Leu Asp Gly Gln Arg Gly
   210 215 220
   Lys Cys Trp Cys Val Asp Arg Lys Thr Gly Val Lys Leu Pro Gly Gly
   225 230 235 240
   Leu Glu Pro Lys Gly Glu Leu Asp Cys His Gln Leu Ala Asp Ser Phe
   245 250 255
   Arg Glu Val Asp Pro Trp Ser His Pro Gln Phe Glu Lys Thr Gly His
   260 265 270
   His His His His His His His Gly Gly Gln
   275 280
<210> 6
   <211> 146
   <212> PRT
   <213> artificial
<220>
   <223> C-terminal fragment of IGFBP-4
<400> 6
   Met Leu Leu Leu Leu Leu Leu Leu Gly Leu Arg Leu Gln Leu Ser Leu
   1 5 10 15
   Gly Ile Ala Ser Lys Val Asn Gly Ala Pro Arg Glu Asp Ala Arg Pro
   20 25 30
   Val Pro Gln Gly Ser Cys Gln Ser Glu Leu His Arg Ala Leu Glu Arg
   35 40 45
   Leu Ala Ala Ser Gln Ser Arg Thr His Glu Asp Leu Tyr Ile Ile Pro
   50 55 60
   Ile Pro Asn Cys Asp Arg Asn Gly Asn Phe His Pro Lys Gln Cys His
   65 70 75 80
   Pro Ala Leu Asp Gly Gln Arg Gly Lys Cys Trp Cys Val Asp Arg Lys
   85 90 95
   Thr Gly Val Lys Leu Pro Gly Gly Leu Glu Pro Lys Gly Glu Leu Asp
   100 105 110
   Cys His Gln Leu Ala Asp Ser Phe Arg Glu Val Asp Pro Trp Ser His
   115 120 125
   Pro Gln Phe Glu Lys Thr Gly His His His His His His His His Gly
   130 135 140
   Gly Gln
   145
<210> 7
   <211> 296
   <212> PRT
   <213> human
<400> 7
   Met Val Leu Leu Thr Ala Val Leu Leu Leu Leu Ala Ala Tyr Ala Gly
   1               5                   10                  15
   Pro Ala Gln Ser Leu Gly Ser Phe Val His Cys Glu Pro Cys Asp Glu
               20                  25                  30
   Lys Ala Leu Ser Met Cys Pro Pro Ser Pro Leu Gly Cys Glu Leu Val
           35                  40                  45
   Lys Glu Pro Gly Cys Gly Cys Cys Met Thr Cys Ala Leu Ala Glu Gly
       50                  55                  60
   Gln Ser Cys Gly Val Tyr Thr Glu Arg Cys Ala Gln Gly Leu Arg Cys
   65                  70                  75                  80
   Leu Pro Arg Gln Asp Glu Glu Lys Pro Leu His Ala Leu Leu His Gly
                   85                  90                  95
   Arg Gly Val Cys Leu Asn Glu Lys Ser Tyr Arg Glu Gln Val Lys Ile
               100                 105                 110
   Glu Arg Asp Ser Arg Glu His Glu Glu Pro Thr Thr Ser Glu Met Ala
           115                 120                 125
   Glu Glu Thr Tyr Ser Pro Lys Ile Phe Arg Pro Lys His Thr Arg Ile
       130                 135                 140
   Ser Glu Leu Lys Ala Glu Ala Val Lys Lys Asp Arg Arg Lys Lys Leu
   145                 150                 155                 160
   Thr Gln Ser Lys Phe Val Gly Gly Ala Glu Asn Thr Ala His Pro Arg
                   165                 170                 175
   Ile Ile Ser Ala Pro Glu Met Arg Gln Glu Ser Glu Gln Gly Pro Cys
               180                 185                 190
   Arg Arg His Met Glu Ala Ser Leu Gln Glu Leu Lys Ala Ser Pro Arg
           195                 200                 205
   Met Val Pro Arg Ala Val Tyr Leu Pro Asn Cys Asp Arg Lys Gly Phe
       210                 215                 220
   Tyr Lys Arg Lys Gln Cys Lys Pro Ser Arg Gly Arg Lys Arg Gly Ile
   225                 230                 235                 240
   Cys Trp Cys Val Asp Lys Tyr Gly Met Lys Leu Pro Gly Met Glu Tyr
                   245                 250                 255
   Val Asp Gly Asp Phe Gln Cys His Thr Phe Asp Ser Ser Asn Val Glu
               260                 265                 270
   Gly Ser Gly Trp Ser His Pro Gln Phe Glu Lys Thr Gly His His His
           275                 280                 285
   His His His His His Gly Gly Gln
       290                 295
<210> 8
   <211> 139
   <212> PRT
   <213> artificial
<220>
   <223> C-terminal fragment of IGFBP-5
<400> 8
   Met Leu Leu Leu Leu Leu Leu Leu Gly Leu Arg Leu Gln Leu Ser Leu
   1 5 10 15
   Gly Ile Ala Ser Ile Ile Ser Ala Pro Glu Met Arg Gln Glu Ser Glu
   20 25 30
   Gln Gly Pro Cys Arg Arg His Met Glu Ala Ser Leu Gln Glu Leu Lys
   35 40 45
   Ala Ser Pro Arg Met Val Pro Arg Ala Val Tyr Leu Pro Asn Cys Asp
   50 55 60
   Arg Lys Gly Phe Tyr Lys Arg Lys Gln Cys Lys Pro Ser Arg Gly Arg
   65 70 75 80
   Lys Arg Gly Ile Cys Trp Cys Val Asp Lys Tyr Gly Met Lys Leu Pro
   85 90 95
   Gly Met Glu Tyr Val Asp Gly Asp Phe Gln Cys His Thr Phe Asp Ser
   100 105 110
   Ser Asn Val Glu Asp Pro Trp Ser His Pro Gln Phe Glu Lys Thr Gly
   115 120 125
   His His His His His His His His Gly Gly Gln
   130 135
<210> 9
   <211> 240
   <212> PRT
   <213> human
<400> 9
   Met Thr Pro His Arg Leu Leu Pro Pro Leu Leu Leu Leu Leu Ala Leu
   1               5                   10                  15
   Leu Leu Ala Ala Ser Pro Gly Gly Ala Leu Ala Arg Cys Pro Gly Cys
               20                  25                  30
   Gly Gln Gly Val Gln Ala Gly Cys Pro Gly Gly Cys Val Glu Glu Glu
           35                  40                  45
   Asp Gly Gly Ser Pro Ala Glu Gly Cys Ala Glu Ala Glu Gly Cys Leu
       50                  55                  60
   Arg Arg Glu Gly Gln Glu Cys Gly Val Tyr Thr Pro Asn Cys Ala Pro
   65                  70                  75                  80
   Gly Leu Gln Cys His Pro Pro Lys Asp Asp Glu Ala Pro Leu Arg Ala
                   85                  90                  95
   Leu Leu Leu Gly Arg Gly Arg Cys Leu Pro Ala Arg Ala Pro Ala Val
               100                 105                 110
   Ala Glu Glu Asn Pro Lys Glu Ser Lys Pro Gln Ala Gly Thr Ala Arg
           115                 120                 125
   Pro Gln Asp Val Asn Arg Arg Asp Gln Gln Arg Asn Pro Gly Thr Ser
       130                 135                 140
   Thr Thr Pro Ser Gln Pro Asn Ser Ala Gly Val Gln Asp Thr Glu Met
   145                 150                 155                 160
   Gly Pro Cys Arg Arg His Leu Asp Ser Val Leu Gln Gln Leu Gln Thr
                   165                 170                 175
   Glu Val Tyr Arg Gly Ala Gln Thr Leu Tyr Val Pro Asn Cys Asp His
               180                 185                 190
   Arg Gly Phe Tyr Arg Lys Arg Gln Cys Arg Ser Ser Gln Gly Gln Arg
           195                 200                 205
   Arg Gly Pro Cys Trp Cys Val Asp Arg Met Gly Lys Ser Leu Pro Gly
       210                 215                 220
   Ser Pro Asp Gly Asn Gly Ser Ser Ser Cys Pro Thr Gly Ser Ser Gly
   225                 230                 235                 240
<210> 10
   <211> 125
   <212> PRT
   <213> artificial
<220>
   <223> C-terminal fragment of IGFBP-6
<400> 10
   Met Gly Glu Leu Leu Leu Leu Leu Leu Leu Gly Leu Arg Leu Gln Leu
   1               5                   10                  15
   Ser Leu Gly Ile Ala Arg Asn Ser Ala Gly Val Gln Asp Thr Glu Met
               20                  25                  30
   Gly Pro Cys Arg Arg His Leu Asp Ser Val Leu Gln Gln Leu Gln Thr
           35                  40                  45
   Glu Val Tyr Arg Gly Ala Gln Thr Leu Tyr Val Pro Asn Cys Asp His
       50                  55                  60
   Arg Gly Phe Tyr Arg Lys Arg Gln Cys Arg Ser Ser Gln Gly Gln Arg
   65                  70                  75                  80
   Arg Gly Pro Cys Trp Cys Val Asp Arg Met Gly Lys Ser Leu Pro Gly
                   85                  90                  95
   Ser Pro Asp Gly Asn Gly Ser Ser Ser Cys Pro Thr Gly Ser Ser Gly
               100                 105                 110
   Ser Ser Thr Gly His His His His His His His His Gly
           115                 120                 125
<210> 11
   <211> 291
   <212> PRT
   <213> human
<400> 11
   Met Gln Arg Ala Arg Pro Thr Leu Trp Ala Ala Ala Leu Thr Leu Leu
   1               5                   10                  15
   Val Leu Leu Arg Gly Pro Pro Val Ala Arg Ala Gly Ala Ser Ser Ala
               20                  25                  30
   Gly Leu Gly Pro Val Val Arg Cys Glu Pro Cys Asp Ala Arg Ala Leu
           35                  40                  45
   Ala Gln Cys Ala Pro Pro Pro Ala Val Cys Ala Glu Leu Val Arg Glu
       50                  55                  60
   Pro Gly Cys Gly Cys Cys Leu Thr Cys Ala Leu Ser Glu Gly Gln Pro
   65                  70                  75                  80
   Cys Gly Ile Tyr Thr Glu Arg Cys Gly Ser Gly Leu Arg Cys Gln Pro
                   85                  90                  95
   Ser Pro Asp Glu Ala Arg Pro Leu Gln Ala Leu Leu Asp Gly Arg Gly
               100                 105                 110
   Leu Cys Val Asn Ala Ser Ala Val Ser Arg Leu Arg Ala Tyr Leu Leu
           115                 120                 125
   Pro Ala Pro Pro Ala Pro Gly Asn Ala Ser Glu Ser Glu Glu Asp Arg
       130                 135                 140
   Ser Ala Gly Ser Val Glu Ser Pro Ser Val Ser Ser Thr His Arg Val
   145                 150                 155                 160
   Ser Asp Pro Lys Phe His Pro Leu His Ser Lys Ile Ile Ile Ile Lys
                   165                 170                 175
   Lys Gly His Ala Lys Asp Ser Gln Arg Tyr Lys Val Asp Tyr Glu Ser
               180                 185                 190
   Gln Ser Thr Asp Thr Gln Asn Phe Ser Ser Glu Ser Lys Arg Glu Thr
           195                 200                 205
   Glu Tyr Gly Pro Cys Arg Arg Glu Met Glu Asp Thr Leu Asn His Leu
       210                 215                 220
   Lys Phe Leu Asn Val Leu Ser Pro Arg Gly Val His Ile Pro Asn Cys
   225                 230                 235                 240
   Asp Lys Lys Gly Phe Tyr Lys Lys Lys Gln Cys Arg Pro Ser Lys Gly
                   245                 250                 255
   Arg Lys Arg Gly Phe Cys Trp Cys Val Asp Lys Tyr Gly Gln Pro Leu
               260                 265                 270
   Pro Gly Tyr Thr Thr Lys Gly Lys Glu Asp Val His Cys Tyr Ser Met
           275                 280                 285
   Gln Ser Lys
       290
<210> 12
   <211> 32
   <212> DNA
   <213> artificial
<220>
   <223> PCR primer for igfbp-1 cloning
<400> 12
   ctagaattcc accatgtcag aggtccccgt tg 32
<210> 13
   <211> 31
   <212> DNA
   <213> artificial
<220>
   <223> pcr primer for igfbp-1 cloning
<400> 13
   ctaaccggtg ttttgtacat taaaatatat c 31
<210> 14
   <211> 30
   <212> DNA
   <213> artificial
<220>
   <223> pcr primer for igfbp-2 cloning
<400> 14
   ctagaattcc accatgctgc cgagagtggg 30
<210> 15
   <211> 28
   <212> DNA
   <213> artificial
<220>
   <223> pcr primer for igfbp-2 clone
<400> 15
   tagggatccc tgcatccgct gggtgtgc 28
<210> 16
   <211> 27
   <212> DNA
   <213> artificial
<220>
   <223> pcr primer for IGFBP-2 c-terminal cloning
<400> 16
   ctagctagca agggtggcaa gcatcac 27
<210> 17
   <211> 28
   <212> DNA
   <213> artificial
<220>
   <223> pcr primer for cloning IGFBP-2 c terminal fragment
<400> 17
   tagggatccc tgcatccgct gggtgtgc 28
<210> 18
   <211> 32
   <212> DNA
   <213> artificial
<220>
   <223> pcr primer for igfbp-4 cloning
<400> 18
   taagaattcg ccaccatgct gcccctctgc ct 32
<210> 19
   <211> 30
   <212> DNA
   <213> artificial
<220>
   <223> pcr primer for igfbp-4 cloning
<400> 19
   ttaggatcca cctctcgaaa gctgtcagcc 30
<210> 20
   <211> 32
   <212> DNA
   <213> artificial
<220>
   <223> pcr primer for cloning igfbp-4 c-terminal clone
<400> 20
   gccgctagca aggtcaatgg ggcgccccgg ga 32
<210> 21
   <211> 30
   <212> DNA
   <213> artificial
<220>
   <223> pcr primer for igfbp-4 c-terminal clone
<400> 21
   ttaggatcca cctctcgaaa gctgtcagcc 30
<210> 22
   <211> 34
   <212> DNA
   <213> artificial
<220>
   <223> pcr primer for igfbp-5 clone
<400> 22
   ctagaattcc accatggtgt tgctcaccgc ggtc 34
<210> 23
   <211> 33
   <212> DNA
   <213> artificial
<220>
   <223> pcr primer for igfbp-5 clone
<400> 23
   ctaggatccc tcaacgttgc tgctgtcgaa ggt 33
<210> 24
   <211> 30
   <212> DNA
   <213> artificial
<220>
   <223> pcr primer for igfbp-5 c terminal clone
<400> 24
   ctagctagca tcatctctgc acctgagatg 30
<210> 25
   <211> 33
   <212> DNA
   <213> artificial
<220>
   <223> pcr primer for igfbp-5 c-terminal clone
<400> 25
   ctaggatccc tcaacgttgc tgctgtcgaa ggt 33

## Description

### FIELD OF THE INVENTION

The present invention relates to methods for inhibiting angiogenesis, tumorigenesis and cathepsin activity, particularly in mammalian cells.

### BACKGROUND OF THE INVENTION

The insulin-like growth factor binding protein (IGFBP) family comprises six related proteins (IGFBP1-6) that interact with high affinity with insulin-like growth factors (IGFs) and modulate their biological effects. In circulation and interstitial fluids, IGFBPs are the major carrier proteins for IGFs and prevent their degradation by proteases. IGFs can only bind to IGF surface receptors after IGFBP proteolysis. By sequestering IGFs away from IGF receptors, IGFBPs inhibit mitogenesis, differentiation, survival and other IGF-induced events. IGFBPs also have IGF-independent effects on different cell types, although the mechanism(s) of action are still unknown.

Sequence analyses of the IGFBP protein family indicate the presence of a conserved thyroglobulin type-1 domain in all family members. Proteins bearing type-1 domains have been shown to inhibit cysteine proteinase(s) (cathepsins). Cathepsins are proteases, normally present in lysosomes, which play an important role in many physiological processes such as protein degradation, antigen presentation, and bone resorption. In tumors and activated cells, cathepsins can be translocated to the membrane and secreted to extracellular spaces, participating in degradation of extracellular matrix (ECM), facilitating in this manner cell migration. Tumor invasion, angiogenesis and metastasis have been associated with altered lysosomal trafficking and increased expression of lysosomal cathepsins. More specifically, the lysosomal cysteine protease cathepsin B has been recently implicated in tumor dissemination and angiogenesis. The proteolytic activity of cathepsin B facilitates direct degradation of various ECM proteins, including laminin, fibronectin, tenascin C, and type IV collagen, the latter being a major component of ECM and the vascular basement membrane. Cathepsin B has also been implicated in the activation of other enzymes of the proteolytic cascade mediating ECM degradation, such as metalloproteases and urokinase plasminogen activator (uPA). Cathepsin B is present in the lysosomes of various cell types, including endothelial cells. Recent studies have shown that, in tumor and endothelial cells, both extracellular and, more significantly, intracellular cathepsin B are involved in ECM degradation. The presence of cathepsin B in endothelial cells of brain tumors correlates with poor survival of these patients and can therefore be used as a prognostic indicator.

Commonly held copending PCT application PCT/CA2006/000250 filed February 20, 2006discloses that IGFBP-4 (IBP-4) is a potent and pleiotropic anti-angiogenic and anti-tumorigenic factor. In particular, this copending application discloses that the C-terminal protein fragment of IGFBP-4 (CIBP-4), which contain a thyroglobulin type I (TY) domain, has anti-angiogenic activity.

The documents WO2006034832 and WO0023469 thereby describe the use of IGFBP-2.

### Summary of the Invention

It has now been found that insulin-like growth factor binding proteins (IGFBPs) and variants thereof inhibit cathepsin activity.

In one aspect of the invention, there is provided a use of an insulin-like growth factor binding protein or a variant thereof for inhibiting cathepsin activity.

It is of note that as used herein, 'variants' also includes modifications such as PEGylation, glycosylation, cyclation or derivitivization of one or more functional groups.

It is further of note that he homology of the IGFBP sequences varies between 54-70%. For the C-terminal fragments that we have produced the homology varies between 54-82%, as discussed below.

In a first aspect of the invention, there is provided the use of a peptide consisting of an amino acid sequence with at least 85% identity to amino acids 1-328 of SEQ ID No. 3 or amino acids 107-328 of SEQ ID No. 3 in the preparation of a medicament for inhibiting angiogenesis.

In a second aspect of the invention, there is provided the use of a peptide consisting of an amino acid sequence with at least 85% identity to amino acids 1-328 of SEQ ID No. 3 or amino acids 107-328 of SEQ ID No. 3 in the preparation of a medicament for inhibiting cathepsin activity.

In a third aspect of the invention, there is provided the use of a peptide consisting of amino acids 1-328 of SEQ ID No. 3 or amino acids 107-328 of SEQ ID No. 3 in the preparation of a medicament for inhibiting angiogenesis.

In a fourth aspect of the invention, there is provided the use of a peptide consisting of amino acids 1-328 of SEQ ID No. 3 or amino acids 107-328 of SEQ ID No. 3 in the preparation of a medicament for inhibiting cathepsin activity.

In a fifth aspect of the invention, there is provided the use of a peptide comprising an amino acid sequence with at least 85% identity to amino acids 1-328 of SEQ ID No. 3 or amino acids 107-328 of SEQ ID No. 3 in the preparation of a medicament for inhibiting cathepsin activity.

In a sixth aspect of the invention, there is provided the use of a peptide comprising amino acids 1-328 of SEQ ID No. 3 or amino acids 107-328 of SEQ ID No. 3 in the preparation of a medicament for inhibiting cathepsin activity.

Insulin-like growth factor binding proteins (IGFBPs) include, for example, IGFBP-1, IGFBP-2, IGFBP-3, IGFBP-4, IGFBP-5, IGFBP-6 or a mixture thereof. Variants of such proteins are preferably the C-terminal protein fragments of the IGFBPs, particularly the thyroglobulin type I (TY) domain in the C-terminal fragments. In some cases, variants preferably have an amino acid sequence having at least 70% sequence identity to one of the IGFBPs, preferably at least 75%, 80%, 85%, 90%, 95%, 97%, 98% or 99% sequence identity. In some cases, variants preferably have an amino acid sequence having at least 70% sequence identity to one of the C-terminal fragments of an IGFBP, preferably at least 75%, 80%, 85%, 90%, 95%, 97%, 98% or 99% sequence identity.

In other embodiments, the IGFBP peptides comprise or consist or consist essentially of the TY1 domains, that is, comprise, consist or consist essentially of amino acids corresponding to amino acids 173-251 of SEQ ID No. 1 (IGFBP-1), amino acids 207-309 of SEQ ID No. 3 (IGFBP-2), amino acids 210-285 of SEQ ID No. 11 (IGFBP-3), amino acids 171-249 of SEQ ID No. 5 (IGFBP-4), amino acids 189-263 of SEQ ID No. 7 (IGFBP-5) or amino acids 160-234 of SEQ ID No. 9 (IGFBP-6).

The IGFBP proteins and fragments thereof inhibit cathepsin activity, particularly cathepsin B activity, and thus are useful as active agents to delay or prevent acute or chronic disease states associated with cathepsin activity. Such disease states include, for example, neurodegenerative disorders including ischemic stroke (thrombotic or embolic in origin), hemmorhagic stroke and subsequent vascular phenomena, myocardial infarction, neurologic consequences of coronary bypass and grafting operations, head trauma, Alzheimer's Disease, age-associated dementia, vascular dementias, Parkinson's disease and amyotrophic lateral sclerosis. The IGFBP proteins and fragments thereof are particularly useful for inhibiting angiogenesis and/or tumorigenesis. The IGFBP proteins and fragments thereof are particularly useful in mammals, particularly in mammalian cells.

As will be appreciated by one of skill in the art, proteases have the potential to cause significant tissue damage due to the hydrolysis of a wide variety of intracellular and extracellular substrates. Uncontrolled release of proteases can exacerbate the ongoing tissue damage initiated by primary mechanical injury. Lysosomal leakage or rupture with the subsequent release of proteases represents the greatest threat to neuronal survival. Abnormal increase in cathepsin B activity intra or extracellularly can affect protein degradation and cellular integrity. Cathepsin B has been recently associated with neuronal cell death and apoptosis. Upregulation of cathepsin B has been reported to occur in multiple neurodegenerative disorders including stroke, Alzheimer's disease, head trauma, dementia and the like.

In a use or method of treatment in effecting treatment of a patient afflicted with a disease state described above, IGFPBs or fragments thereof can be administered in any form or mode which makes them bioavailable in effective amounts, including oral and parenteral routes. For example, IGFPBs or fragments thereof can be administered orally, subcutaneously, intramuscularly, intravenously, transdermally, intranasally, rectally, topically, and the like. Oral or intravenous administration is generally preferred. One skilled in the art of preparing formulations can readily select the proper form and mode of administration depending upon the particular characteristics of the active agent selected for the disease state to be treated, the stage of the disease, and other relevant circumstances. Remington's Pharmaceutical Sciences, 18th Edition, Mack Publishing Co. (1990). It is of note that 'an effective amount' may be approximately 0.1-30 mg/kg, depending of course on the age, weight and condition of the patient as well as on the delivery method chosen.

The active agents may be formulated as a medicament and can be administered alone or in the form of a pharmaceutical composition in combination with pharmaceutically acceptable carriers or excipients, the proportion and nature of which are determined by the solubility and chemical properties of the active agent selected, the chosen route of administration, and standard pharmaceutical practice.

Further features of the invention will be described or will become apparent in the course of the following detailed description.

### Brief Description of the Drawings

In order that the invention may be more clearly understood, embodiments thereof will now be described in detail by way of example, with reference to the accompanying drawings, in which:

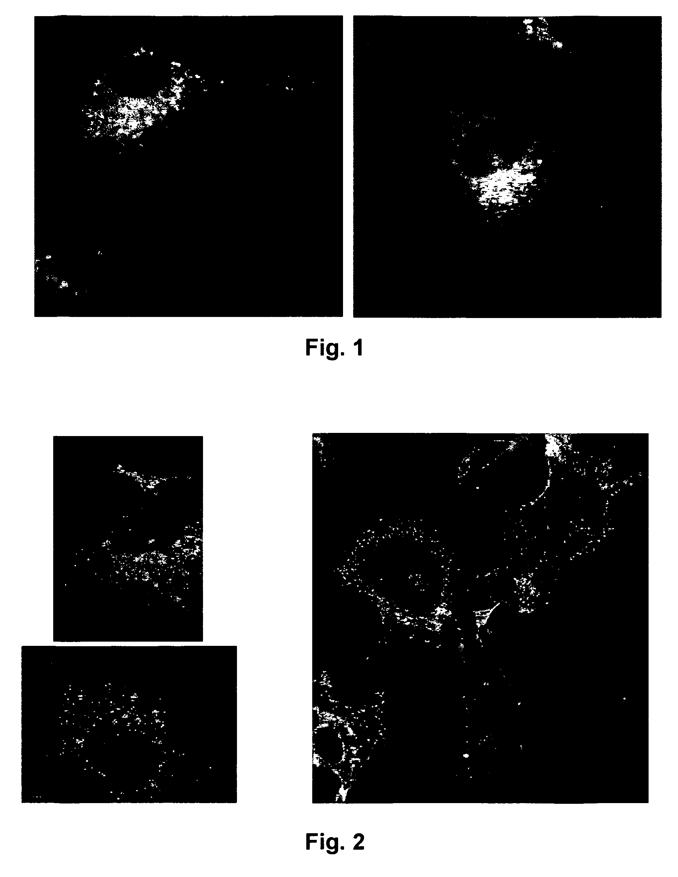

Fig. 1 depicts confocal microscopy images showing internalization of CIBP4 (orange) in human brain endothelial cells (stained with the membrane dye DiCO3(5), green) targeting perinuclear lysosomal-like structures.
Fig. 2 depicts confocal microscopy images showing co-localizaton (pink) of CIBP4 (orange) and lysosomes (stained with Lysotracker™ solution, blue) in HBEC (stained with a membrane dye DiCO3(5), green).

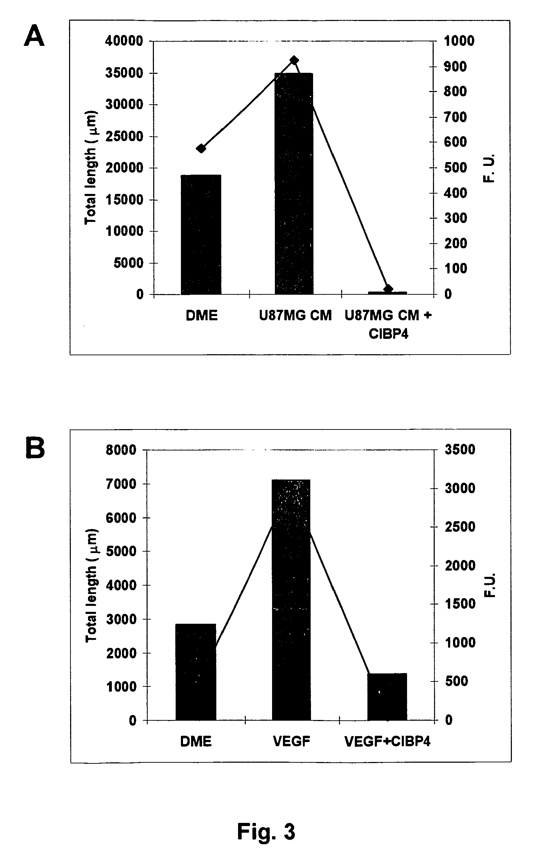

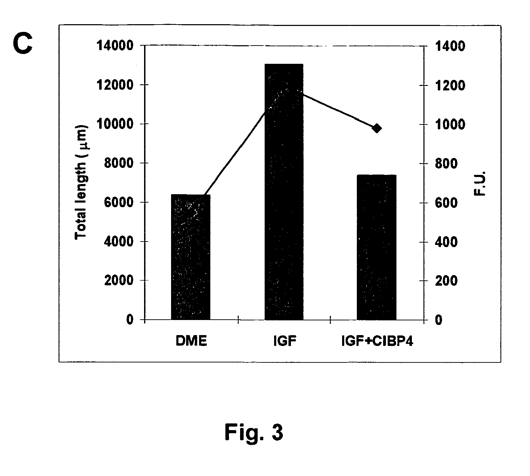

Fig. 3 depicts representative experiments in which bar graphs (left hand axis) indicate the total length of the capillary-like tubes (CLT) formed overnight by HBEC seeded on Matrigel (*in vitro* angiogenesis assay) and exposed to DME (**A**-**C**) or to proangiogenic stimuli (U87MG CM, A; VEGF, **B**; IGF-1, **C**) either alone or in combination with 20 nM CIBP4 (**A-C**). Lines (right hand axis) indicate the levels of intracellular cathepsin B activity (measured as fluorescence units, F.U., after incubation with Magic Red™ Cathepsin B detection solution for 2h) in HBEC at the end of the experiment. Similar correlation pattern between angiogenesis and cathepsin B activity was obtained in 2-3 additional experiments.

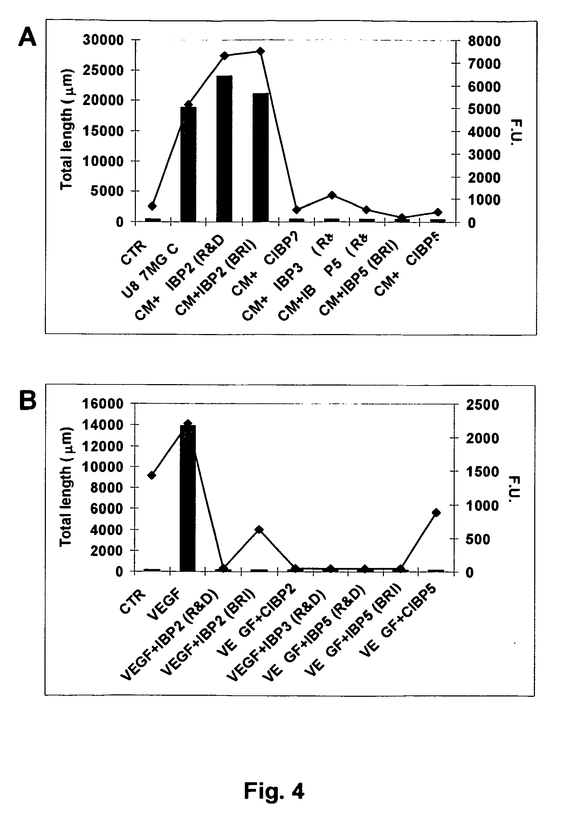

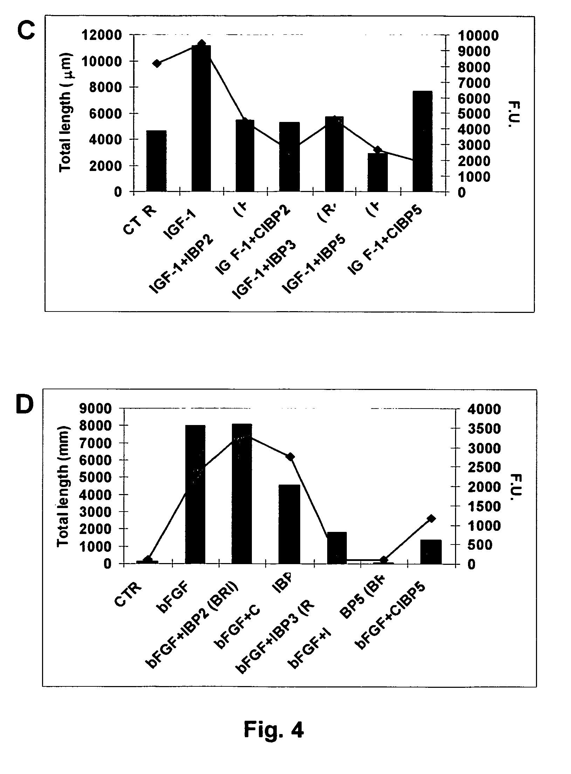

Fig. 4 depicts representative experiments in which bar graphs (left hand axis) indicate the total length of the CLT formed overnight by HBEC seeded on Matrigel and exposed to DME (**A**-**D**) or to proangiogenic stimuli (U87MG CM, **A**; VEGF, **B**; IGF, **C**; bFGF, **D**) either alone or in combination with 20 nM IBP-2 (from R&D systems), 20 nM IBP-2 (produced at NRC), 20 nM CBP-2, 20 nM IBP-3 (from R&D systems), 20 nM IBP-5 (from R&D systems), 20 nM IBP-5 (produced by NRC) or 20 nM CIBP5 (**A-D**). Lines (right hand axis) indicate the levels of intracellular cathepsin B activity, (measured as fluorescence units, F.U) in HBEC at the end of the experiment. Similar correlation pattern between angiogenesis and cathepsing B activity was obtained in 2-3 additional experiments

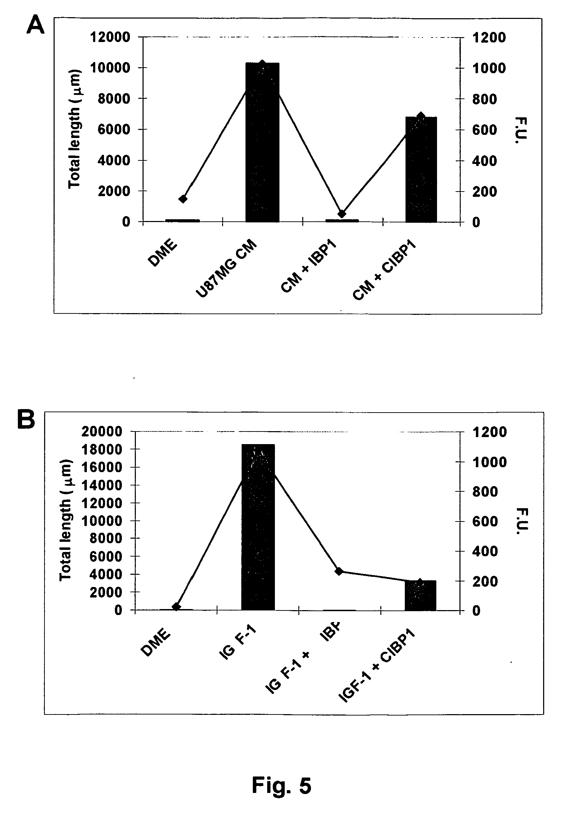

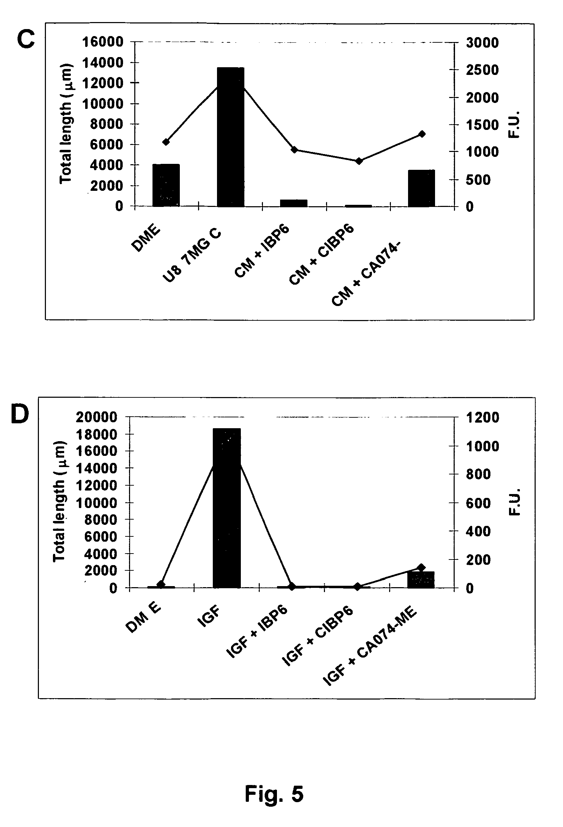

Fig. 5 depicts representative experiments in which bar graphs (left hand axis) represent the total length of the CLT formed overnight by HBEC seeded on Matrigel and exposed to DME (**A-D**) or to proangiogenic stimuli (U87MG CM, **A**&**C**; IGF-1, **B**&**D**) either alone or in combination with 20 nM IBP-1 and 20 nM CIBP-1 (both produced at BRI-NRC) (**A-B**) or 20 nM IBP-6 (from R&D systems) and 20 nM CIBP-6 (produced at NRC) (**C-D**). Lines (right hand axis) indicate the levels of intracellular cathepsin B activity (measured as fluorence units F.U) in HBEC at the end of the experiment. Similar correlation pattern between angiogenesis and cathepsing B activity was obtained in 2-3 additional experiments

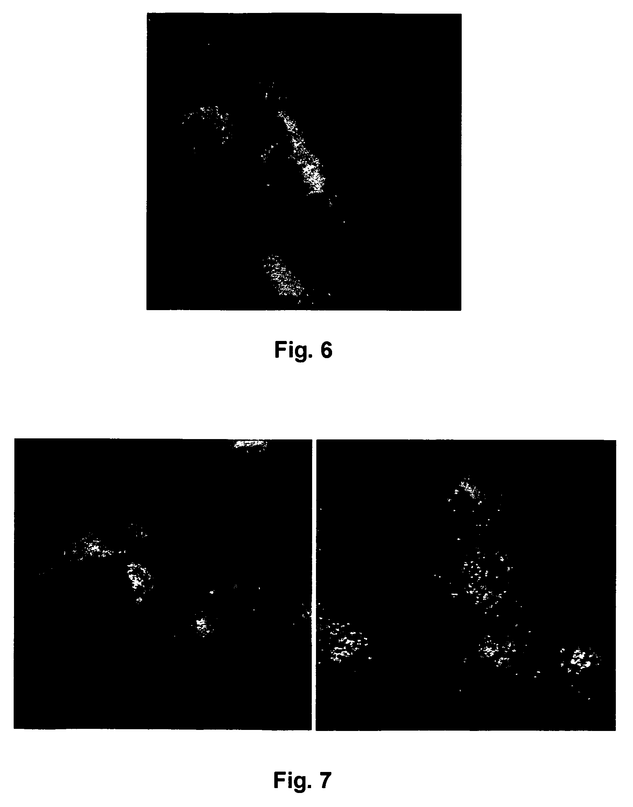

Fig. 6 depicts confocal microscopy images showing cellular distribution of cathepsin B activity (blue) in U87MG cells (stained with a membrane dye, green) after 15 min incubation with Magic Red™Cathepsin B detection reagent.
Fig. 7 depicts confocal microscopy images showing co-localizaton (pink) of CIBP4 (orange) and lysosomes (stained with Lysotracker™ solution, blue) in U87MG cells (stained with a membrane dye DiCO3(5), green).

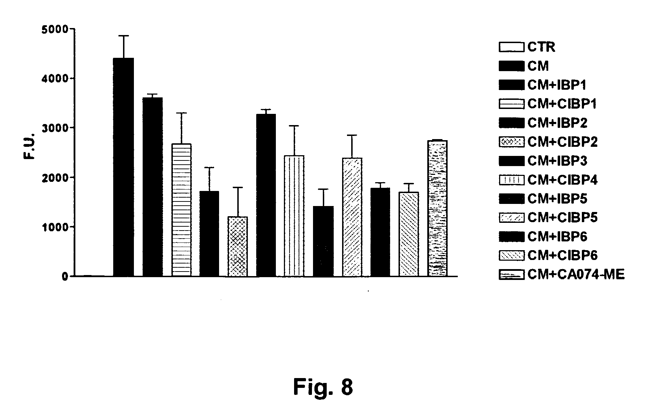

Fig. 8 depicts cathepsin B activity measured in DME (control) and in U87MG CM untreated or treated overnight with 20 nM of either IBP-1, CIBP1, IBP2, CIBP2, IBP3, CIBP4, IBP5, CIBP5, IBP6, CIBP6 or 10 µM CA074-ME, a synthetic permeable cathepsin B inhibitor (EMD Biosciences, Canada) Bars are means ± s.e.m. of two experiments done in triplicate

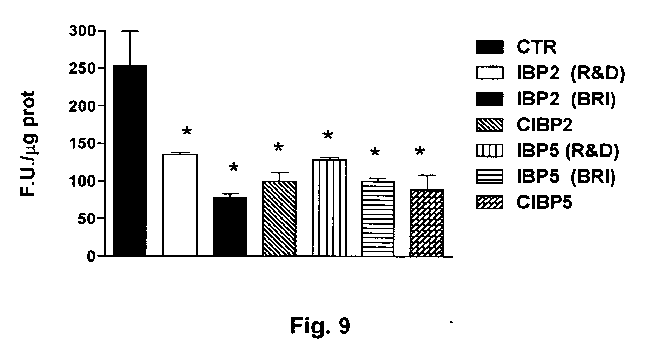

Fig. 9. depicts intracellular cathepsin B activity measured in U87MG cells exposed overnight to DME (control) or to 20 nM IBP2 (from R&D systems), IBP2 (produced at BRI-NRC), CIBP2, IBP5 (from R&D systems), IBP5 (produced at BRI-NRC) and CIBP5. Bars are means ± s.e.m. of two experiments done in triplicate. * indicates significance (p<0.05, ANOVA followed by Newman-Keuls) between U87MG cells exposed to DME and those exposed to IGFBP members and fragments.

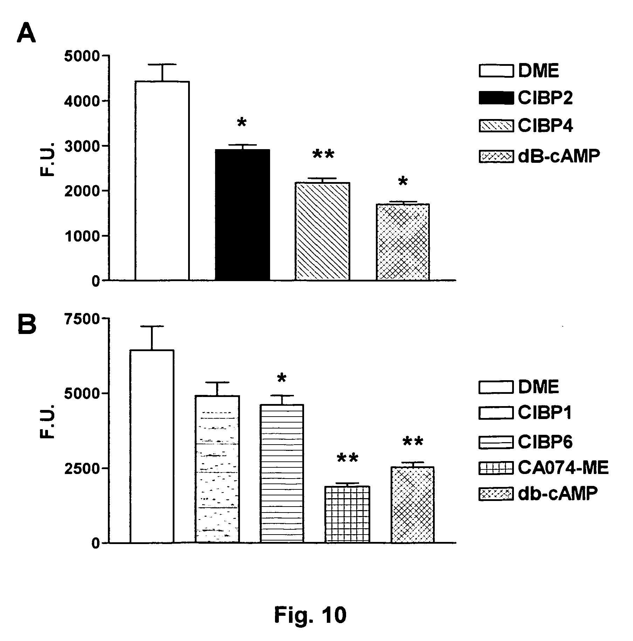

Fig. 10 depicts the anchorage-dependent growth (assayed by Alamar Blue fluorescence measurement) of U87MG cells in soft agar in the absence (control) or presence of 20 nM of either CIBP2, CIBP4, or 500 µg/ml dB-cAMP (A) and 20 nM CIBP1, CIBP6, 10 µM of CA074-ME or 500 µg/ml dB-cAMP (B). Bars are means ± s.e.m. of two experiments done in triplicate. * indicates significance (p<0.05, ANOVA followed by Newman-Keuls) between control and treatments.

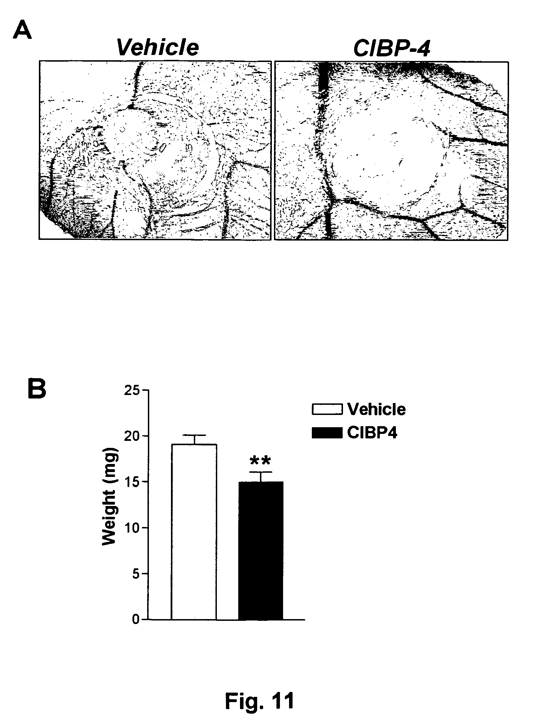

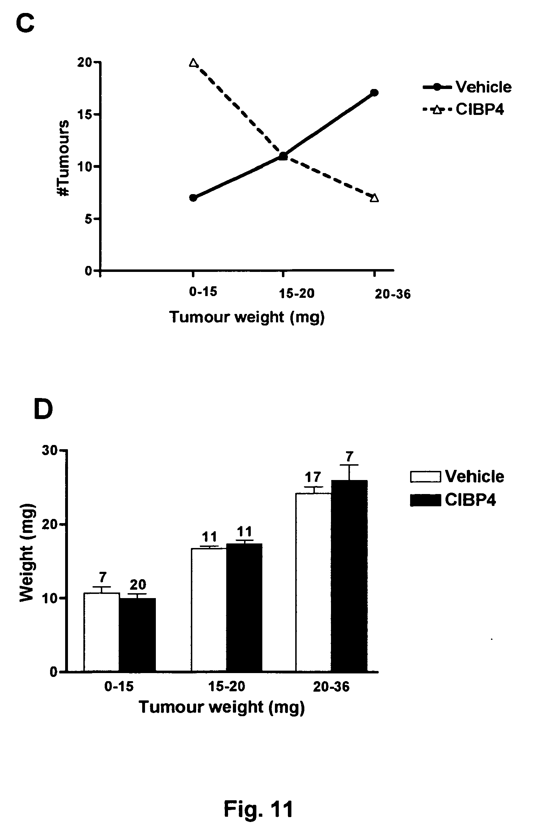

Fig. 11 depicts a representative image of tumors formed by the growth of U87MG cells on the chick chorioallantoic membrane (CAM) of fertilized eggs treated for 4 consecutive days with vehicle (A, left panel) or 250 nM CIBP-4 (A, right panel). Tumor weight was evaluated after treatment with vehicle or 250 nM CIBP-4 (B). Bars are means ± s.e.m. of 35 (vehicle) and 38 (CIBP4) eggs.** indicates significance (p<0.005, unpaired t-test) between vehicle- and CIBP4-treated tumors. Frequency distribution of tumor weight in three intervals (0-15 mg, 15-20 mg, 20-36 mg) was analyzed (C) Average weight of tumors in each interval was evaluated in both vehicle- (grey bars) and CIBP4- (black bars) treated groups (D). Numbers on the bars indicate the number of tumors that belong to the corresponding weight interval in each group

### Description of Preferred Embodiments

Unless defined otherwise, all technical and scientific terms used herein have the same meaning as commonly understood by one of ordinary skill in the art to which the invention belongs. Although any methods and materials similar or equivalent to those described herein can be used in the practice or testing of the present invention, the preferred methods and materials are now described. All publications mentioned hereunder are incorporated herein by reference.

In an embodiment of the invention there is provided a method of reducing angiogenesis by modulating the interaction of IGF with a receptor, comprising regulating the concentration of IGFBP-1, IGFBP-2, IGFBP-3, IGFBP-4, IGFBP-5 and/or IGFBP-6 in the vicinity of the receptor. In some embodiments, the concentration of IGFBP-1, IGFBP-2, IGFBP-3, IGFBP-5 and/or IGFBP-6 is regulated.

In an embodiment of the invention there is provided an amino acid sequence useful in inhibiting angiogenic responses induced by a variety of growth factors in endothelial cells and/or invasive properties of glioblastoma cells. In some instances, the amino acid sequence is at least 70%, 75%, 80%, 85%, 90%, 95%, 98%, 99% or 100% identical in amino acid sequence to at least one of SEQ ID NO. 1, 2, 3, 4, 5, 6, 7, 8, 9, 10 or 11. In, some instances, differences in amino acid sequence identity will be attributable to conservative substitutions wherein amino acids are replaced by amino acids having a similar size, charge and level of hydrophobicity.

In a preferred embodiment, the angiogenic inhibiting peptide comprises 20 or more consecutive amino acids of any one of: amino acids 1-259 of SEQ ID No. 1 (full length IGFBP-1); amino acids 170-259 of SEQ ID No. 1 (SEQ ID No. 2, C-terminal fragment of IGFBP-1); amino acids 1-328 of SEQ ID No. 3 (full length IGF2); amino acids 107-328 of SEQ ID No. 3 (C-terminal fragment of IGF2, SEQ ID No. 4); amino acids 1-258 of SEQ ID No. 5 (full length IGF4); amino acids 157-258 of SEQ ID No. 5 (C-terminal fragment of IGF4, SEQ ID No. 6); amino acids 1-272 of SEQ ID No. 7 (full length IGF5); amino acids 177-272 (C-terminal fragment of IGF5, SEQ ID No. 8); amino acids 1-240 of SEQ ID No. 9 (full length IGF-6); amino acids 151-240 of SEQ ID No. 9 (C-terminal fragment of IGF-6, SEQ ID No. 10); and amino acids 1-291 of SEQ ID No. 3 (IGFBP-3).

In a further embodiment, the peptide comprises an amino acid sequence that is at least 70%, 75%, 80%, 85%, 90%, 95%, 98%, 99% or 100% identical to amino acids 1-259 of SEQ ID No. 1 (full length IGFBP-1); amino acids 170-259 of SEQ ID No. 1 (SEQ ID No. 2, C-terminal fragment of IGFBP-1); amino acids 1-328 of SEQ ID No. 3 (full length IGFBP-2); amino acids 107-328 of SEQ ID No. 3 (C-terminal fragment of IGFBP-2, SEQ ID No. 4); amino acids 1-258 of SEQ ID No. 5 (full length IGFBP-4); amino acids 157-258 of SEQ ID No. 5 (C-terminal fragment of IGFBP-4, SEQ ID No. 6); amino acids 1-272 of SEQ ID No. 7 (full length IGFBP-5); amino acids 177-272 (C-terminal fragment of IGFBP-5, SEQ ID No. 8); amino acids 1-240 of SEQ ID No. 9 (full length IGFBP-6); amino acids 151-240 of SEQ ID No. 9 (C-terminal fragment of IGFBP-6, SEQ ID No. 10); and amino acids 1-291 of SEQ ID No. 11 (IGFBP-3). As will be appreciated by one of skill in the art, suitable substitutions may be determined by comparing the IGFBP sequence with other IGFBP family members. Specifically, amino acid locations within IGFBPs likely to tolerate substitution are not likely to be highly conserved between IGFBP family members. Furthermore, tolerated conserved substitutions may be determined by comparing the sequences as well. It is of note that as discussed above, the percent of homology of the IGFBP1-6 sequences varies between 54-70%.

In other embodiments, the IGFBP peptide sequence may be flanked on either side or both by additional amino acids which may or may not be 'native' IGFBP sequence or may be within a carrier or presenting peptide as known in the art.

As will be appreciated by one of skill in the art, the C-terminal fragments discussed above represent fragments of native sequence shown to have significant activity. Accordingly, longer fragments, including additional native or in some embodiments non-native amino acids are within the scope of the invention.

In an aspect of the invention there are provided nucleic acid sequences encoding one or more of the amino acid sequences described above.

In an embodiment of the invention there is provided the use of an amino acid sequence having at least 70% sequence identity to amino acids 1-259 of SEQ ID No. 1 (full length IGFBP-1); amino acids 170-259 of SEQ ID No. 1 (SEQ ID No. 2, C-terminal fragment of IGFBP-1); amino acids 1-328 of SEQ ID No. 3 (full length IGFBP-2); amino acids 107-328 of SEQ ID No. 3 (C-terminal fragment of IGFBP-2, SEQ ID No. 4); amino acids 1-258 of SEQ ID No. 5 (full length IGFBP-4); amino acids 157-258 of SEQ ID No. 5 (C-terminal fragment of IGFBP-4, SEQ ID No. 6); amino acids 1-272 of SEQ ID No. 7 (full length IGFBP-5); amino acids 177-272 (C-terminal fragment of IGFBP-5, SEQ ID No. 8); amino acids 1-240 of SEQ ID No. 9 (full length IGFBP-6); amino acids 151-240 of SEQ ID No. 9 (C-terminal fragment of IGFBP-6, SEQ ID No. 10); and amino acids 1-291 of SEQ ID No. 11 (IGFBP-3) to inhibit tumor growth in a mammal. In some cases sequence identity is preferably at least 75%, 80%, 85%, 90%, 95%, 97%, 98%, 99% or 100%. In some cases the sequence includes non-natural and/or chemically modified amino acids.

In an embodiment of the invention there is provided use of an IGFBP peptide or a fragment or variant thereof as described above in modulating the activity of or biological response to one or more growth factors. In some cases the growth factor whose biological activity is modulated is at least one of: IGFBP-I, VEGF and bFGF.

In an embodiment of the invention there is provided a method of inhibiting angiogenic transformation of endothelial cells comprising administering IGFBP-1, IGFBP-2, IGFBP-3, IGFBP-4, IGFBP-5 or IGFBP-6 or a fragment or variant thereof as described above. As discussed above, there are many methods known in the art for measurement of angiogenesis. In some embodiments, inhibition of angiogenesis may be based on a comparison between a treatment group which is administered an effective amount of the IGFBP protein fragment as described herein and an untreated or mock-treated control. It is of note that the control would not necessarily need to be repeated each time.

In some embodiments, the IGFBP peptide as discussed herein may be combined with a matrix, gel or other similar compound such that the IGFBP peptide is substantially retained in a localized area following application thereof to the site of interest.

In an embodiment of the invention there is provided the use of a peptide comprising or consisting of or consisting essentially of amino acids 1-259 of SEQ ID No. 1 (full length IGFBP-1); amino acids 170-259 of SEQ ID No. 1 (SEQ ID No. 2, C-terminal fragment of IGFBP-1); amino acids 1-328 of SEQ ID No. 3 (full length IGFBP-2); amino acids 107-328 of SEQ ID No. 3 (C-terminal fragment of IGFBP-2, SEQ ID No. 4); amino acids 1-258 of SEQ ID No. 5 (full length IGFBP-4); amino acids 157-258 of SEQ ID No. 5 (C-terminal fragment of IGFBP-4, SEQ ID No. 6); amino acids 1-272 of SEQ ID No. 7 (full length IGFBP-5); amino acids 177-272 (C-terminal fragment of IGFBP-5, SEQ ID No. 8); amino acids 1-240 of SEQ ID No. 9 (full length IGFBP-6); amino acids 151-240 of SEQ ID No. 9 (C-terminal fragment of IGFBP-6, SEQ ID No. 10); and amino acids 1-291 of SEQ ID No. 3 (IGFBP-3) or a variant or fragment thereof in the manufacture of a medicament useful for the reduction of angiogenesis or tumor growth or inhibition of cathepsin activity in a mammal. In some instances, the amino acid sequences of the invention may be labeled with radioactive isotopes or fluorescent tags for detection or conjugated to hydrophobic sequences to increase their permeability through biologic membranes.

In some instances, the amino acid sequences of the invention will include non-natural amino acids and/or modified amino acids. Modifications of interest include cyclization, derivitivization and/or glycosylation of one or more functional groups, as discussed above.

In an embodiment of the invention there is provided the use of expression vectors (e.g. bacterial, viral, mammalian, yeast, etc) for generating recombinant protein of one or more of the amino acid sequences described above. As will be appreciated by one skilled in the art, in these embodiments, nucleotide sequences deduced from: amino acids 1-259 of SEQ ID No. 1 (full length IGFBP-1); amino acids 170-259 of SEQ ID No. 1 (SEQ ID No. 2, C-terminal fragment of IGFBP-1); amino acids 1-328 of SEQ ID No. 3 (full length IGFBP-2); amino acids 107-328 of SEQ ID No. 3 (C-terminal fragment of IGFBP-2, SEQ ID No. 4); amino acids 1-258 of SEQ ID No. 5 (full length IGFBP-4); amino acids 157-258 of SEQ ID No. 5 (C-terminal fragment of IGFBP-4, SEQ ID No. 6); amino acids 1-272 of SEQ ID No. 7 (full length IGFBP-5); amino acids 177-272 (C-terminal fragment of IGFBP-5, SEQ ID No. 8); amino acids 1-240 of SEQ ID No. 9 (full length IGFBP-6); amino acids 151-240 of SEQ ID No. 9 (C-terminal fragment of IGFBP-6, SEQ ID No. 10); and amino acids 1-291 of SEQ ID No. 11 may be operably linked to a suitable promoter for expression in the desired host cell.

In other embodiments, the IGFBP peptides comprise or consist or consist essentially of the TY1 domains, that is, comprise, consist or consist essentially of amino acids corresponding to amino acids 173-251 of SEQ ID No. 1 (IGFBP-1), amino acids 207-309 of SEQ ID No. 3 (IGFBP-2), amino acids 210-285 of SEQ ID No. 11 (IGFBP-3), amino acids 171-249 of SEQ ID No. 5 (IGFBP-4), amino acids 189-263 of SEQ ID No. 7 (IGFBP-5) or amino acids 160-234 of SEQ ID No. 9 (IGFBP-6).

In an embodiment of the invention there is provided the use of viral vectors (e.g. retrovirus, adenovirus, adeno-associated virus, herpes-simplex) or non-viral methods of DNA transfer (e.g. naked DNA, liposomes and molecular conjugates, nanoparticles) for delivery and expression of one or more of the amino acid sequences described above in mammalian organs to inhibit pathological angiogenesis or tumor growth or to inhibit cathepsin activity, as discussed herein.

Angiogenesis, the formation of new capillary blood vessels, plays a crucial role in many physiological and pathological settings, including embryonic development, wound healing, ocular diseases, and tumor growth and metastasis. During angiogenesis, new capillaries are formed by a process of sprouting from existing microvessels: in response to locally released angiogenic factors, microvascular endothelial cells degrade their basement membrane and subsequently invade the surrounding interstitial matrix, in which they form tubular capillary sprouts.

Cancer cells are cells that have lost the ability to divide in a controlled fashion. A tumor consists of a population of rapidly dividing and growing cancer cells. Mutations rapidly accrue within the population. These mutations allow the cancer cells to develop drug resistance and escape therapy. Tumors cannot grow beyond a certain size, generally 1-2 mm³, without blood supply due to a lack of oxygen and other essential nutrients. Tumors induce blood vessel growth (angiogenesis) by secreting various growth factors. Growth factors, such as bFGF, IGF-1 and VEGF can induce capillary growth into the tumor, allowing for tumor expansion. Endothelial cells have long been considered genetically more stable than cancer cells. This genomic stability confers an advantage to targeting endothelial cells using antiangiogenic therapy, compared to chemotherapy directed at cancer cells, which rapidly mutate and acquire 'drug resistance' to treatment.

### Materials and Methods:

### Cell Cultures

The human glioma cell line U87MG was established from surgically removed type III glioma/glioblastoma and obtained from ATCC. Cells (5x10⁴cells/ml) were plated in poly-L-lysine pre-coated dishes and grown at 37°C in D-MEM (DME) supplemented with 100 U/ml penicillin, 100 µg/ml streptomycin and 10% heat-inactivated fetal bovine serum (FBS) (HyClone, Logan, Utah) in humidified atmosphere of 5% CO₂/95% air until reached 80% confluence. Then, media were removed and the cells incubated for 3 days in serum-free DME to obtain conditioned media (CM). Conditioned media were collected and filtered (Millex-GV sterilizing filter membrane, 0.22 µm). Cells were then harvested for molecular and biochemical assays.

Human brain endothelial cells (HBEC) were obtained from small intracortical microvessels and capillaries (20-112 µm) harvested from temporal cortex from patients treated surgically for idiopathic epilepsy. Tissues were obtained with approval from the Institutional Research Ethics Committee. HBEC were separated from smooth muscle cells with cloning rings and grown at 37°C in HBEC media containing Earle's salts, 25 mM 4-(2-hydroxyethyl)-1-piperazineethanesulfonic acid (HEPES), 4.35 g/L sodium bicarbonate, and 3 mM L-glutamine, 10% FBS, 5% human serum, 20% of media conditioned by murine melanoma cells (mouse melanoma, Cloudman S91, clone M-3, melanin-producing cells), 5 µg/ml insulin, 5 µg/ml transferrin, 5 ng/ml selenium, and 10 µg/ml endothelial cell growth supplement. HBEC cultures were routinely characterized morphologically and biochemically. More than 95% of cells in culture stained immunopositive for the selective endothelial markers, angiotensin II-converting enzyme and Factor VIII-related antigen, incorporated fluorescently labelled Ac-LDL, and exhibited high activities of the blood-brain barrier- specific enzymes, γ-glutamyltranspeptidase and alkaline phosphatase.

### Production of recombinant full-length IGFBP-1, -2, -3, -4, -5 and -6 and C- terminal protein fragments

### SEQUENCE ID No. 1: Insulin-like growth factor-binding protein 1 precursor (IGFBP-1) (IBP-1/IBP1) (Gene accession number NM 000596.2)

IGFBP-1 gene was amplified by PCR using forward (CTAGAATTCCACCATGTCAGAGGTCCCCGTTG, SEQ ID No. 12) and reverse (CTAACCGGTGTTTTGTACATTAAAATATATC, SEQ ID No. 13) oligos, digested by EcoRI and Agel, and cloned in pTT5SH8Q2 vector. The resulting protein contains a C-terminal octahistidine tag separated from the core protein by a TG linker (see below).

IGFBP-1 full-length protein (aas 1-259)
Normal font: IGFBP-1 amino acid sequence
*Italics: linker+(His)₈GGQ tag*

### SEQUENCE ID No. 2: IGFBP-1 C-terminal domain (CIBP1, Gene accession number AAH57806.1)

CIBP1 gene was codon-optimized and synthesized by GeneScript Corporation digested by Nhel and cloned in Nhel-linearized pTT28 vector. The resulting synthetic gene contains a modified SEAP signal peptide and an octahistidine tag separated from the core protein by an ASSGSSTG linker (see below).

IGFBP-1 C-terminal domain (aa 170-259)
Underlined: Modified Signal Peptide (cleavage predicted between G-I)
Normal font: IGFBP1 C-terminal amino acid sequence (should include IAS residues)
*Italics: linker+(His)₈G tag*

### SEQUENCE ID No. 3: Insulin-like growth factor-binding protein 2 precursor (IGFBP-2) (IBP-1/IBP2) (Gene accession number NM_000597)

IGFBP-2 was amplified by PCR using forward (CTAGAATTCCACCATGCTGCCGAGAGTGGG, SEQ ID No. 14) and reverse (TAGGGATCCCTGCATCCGCTGGGTGTGC, SEQ ID No. 15) oligos, digested by EcoRI and BamHI, and cloned in pYD7SH8Q2 vector. The resulting protein contains a C-terminal Streptagll-octahistidine fusion tag separated from the core protein by a GSG linker (see below).

IGFBP-2 full-length protein (aas 1-328)
Normal font: IGFBP-2 amino acid sequence
*Italics: linker+Streptagll-(His)₈GGQ tag*

### SEQUENCE ID No. 4: IGFBP-2 C-terminal (CIBP2, Gene accession number NM_000597)

CIBP2 was amplified with forward (CTAGCTAGCAAGGGTGGCAAGCATCAC, SEQ ID No. 16) and reverse (TAGGGATCCCTGCATCCGCTGGGTGTGC, SEQ ID No. 17) primers, digested with Nhel and BamHI and cloned in-frame pYD1 vector. The resulting protein contains the SEAP signal peptide and a C-terminal Streptagll-octahistidine fusion tag separated from the core protein by a DP linker (see below).

IGFBP-2 C-terminal fragment (aas 107-328)
Underlined: SEAP Signal Peptide (cleavage predicted between G-I)
Normal font: IGFBP-2 C-terminal amino acid sequence
*Italics: linker+Streptagll-(His)₈GGQ tag*

### SEQUENCE ID No. 5: Insulin-like growth factor binding protein 4 precursor (IGFBP-4) (IBP-4/IBP4) (Gene accession number NP_001543.2)

IGFBP4 was amplified with forward (TAAGAATTCGCCACCATGCTGCCCCTCTGCCT, SEQ ID No. 18) and reverse (TTAGGATCCACCTCTCGAAAGCTGTCAGCC, SEQ ID No. 19) primers, digested with NheI and BamHI and cloned in pTT5SH8Q1 vector. The resulting protein contains a C-terminal Streptagll-octahistidine fusion tag separated from the core protein by a DP linker (see below).

IGFBP-4 full-length protein
Normal font: IGFBP4 amino acid sequence
*Italics: Streptag-II*/*(His)₈G tag (SH8Q1)*

### SEQUENCE ID. No. 6: IGFBP-4 C-terminal (CIBP4, gene Accession number NP_001543.2)

CIBP4 was amplified with forward (GCCGCTAGCAAGGTCAATGGGGCGCCCCGGGA, SEQ ID No. 20) and reverse (TTAGGATCCACCTCTCGAAAGCTGTCAGCC, SEQ ID No. 21) primers, digested with NheI and BamHI and cloned in pYD1 vector. The resulting protein contains the SEAP signal peptide and a C-terminal Streptagll-octahistidine fusion tag separated from the core protein by a DP linker (see below).

IGFBP-4 C-terminal fragment (aas 157-258)
underlined: signal Peptide (SSP)
Normalfont: IGFBP4 C-terminal amino acid sequence (should include IAS residues)
*Italics: Streptag-II*/*(His)₈G tag (SH8Q1)*

### SEQUENCE ID. No. 7: Insulin-like growth factor binding protein 5 (IGFBP-5) (IBP-5/IBP5) (Gene accession number NP_000590.1)

IGFBP-5 was amplified with forward (CTAGAATTCCACCATGGTGTTGCTCACCGCGGTC, SEQ ID No. 22) and reverse (CTAGGATCCCTCAACGTTGCTGCTGTCGAAGGT, SEQ ID No. 23) primers, digested with EcoRI and BamHI and cloned in pTT5SH8Q2 vector. The resulting protein contains the SEAP signal peptide and a C-terminal Streptagll-octahistidine fusion tag separated from the core protein by a GSG linker (see below).

IGFBP-5 full length-protein (aas 1-272)
Normal font: IGFBP5 amino acid sequence
*Italics: Streptag-II*/*(His)₈G tag (SH8Q1)*

### SEQUENCE ID No. 8: IGFBP-5 C-terminal domain (CIBP5, Accession number NP_000590.1)

CIBP5 was amplified with forward (CTAGCTAGCATCATCTCTGCACCTGAGATG, SEQ ID No. 24) and reverse (CTAGGATCCCTCAACGTTGCTGCTGTCGAAGGT, SEQ ID No. 25) primers, digested with Nhel and BamHI and cloned in pYD1 vector. The resulting protein contains the SEAP signal peptide and a C-terminal Streptagll-octahistidine fusion tag separated from the core protein by a DP linker (see below).

IGFBP-5 C-terminal fragment (aas 177-272)
underlined: signal Peptide (SSP)
Normal font: IGFBP5 C-terminal amino acid sequence (should include IAS residues)
*Italics : Streptag-II*/*(His)₈G tag (SH8Q1)*

### SEQUENCE ID No. 9: Insulin-like growth factor binding protein 6 precursor (IGFBP-6) (Accession number NM_002178.2)

### SEQUENCE ID No. 10: Insulin-like growth factor binding protein 6 precursor (IGFBP-6) C-terminal (CIBP6, Accession number NM_002178.2)

CIBP6 was codon-optimized and synthetised by Bio S&T Inc, digested with EcoRI and BamHI and cloned in pTT29 vector. The resulting protein contains a modified SEAP signal peptide and a C-terminal octahistidine tag separated from the core protein by a SSTG linker (see below).

CIBP6 (aas 151-240)
underlined: modified SEAP Signal Peptide
Normal: IGFBP-6 C-terminal aa sequence (should include IAS residues)

### SEQUENCE ID No. 11: Insulin-like growth factor binding protein 3 precursor (IGFBP-3) (Accession number NM_001013398) (amino acids 1-291)

All IGFBP construct were produced following large-scale transfection of HEK293-EBNA1 (293E) cells. Transfection of suspension-growing 293E (clone 6E) cells was done in shaker flasks. Cells were grown in F17 medium (Invitrogen) and transfected at 1x10⁶ cells/ml using 25 kDa linear polyethylenimine as previously described (Durocher et al 2002) with some modifications. For each liter of culture, 750 ug of plasmid DNA was mixed with 1500 ug of PEI and the mixture was incubated for 15 minutes before its addition to the culture. Medium was harvested 5 days later, clarified by filtration through a 0.45 um filter, and loaded on Fractogel cobalt column. The column was washed with Buffer A (50 mM sodium phosphate pH 7.0, 300 mM NaCl), then with Buffer B (Buffer A with 25 mM imidazole) and bound IGFBPs were eluted with Buffer C (Buffer A with 300 mM imidazole). Eluted proteins were then desalted in PBS using a EconoPac 10DG (BioRad) or a HiPrep 26/10 (Pharmacia) column and were sterile-filtered. Protein concentration was estimated by absorbance at 280 nm using a Nanodrop device and their respective molar extinction coefficients.

### CIBP-4 conjugation to Alexa Fluor 647

80 µl of 1 mM Alexa Fluor 647 -NHS in DMSO was added to 0.4 ml of recombinant CIBP-4 (0.2 mg/ml) in 100 mM carbonate pH 8.4, and sample was incubated overnight at room temperature. The reaction was stopped with 150 µl of 200mM ethanolamine pH 8.0. To remove free dye, sample was diluted with 4.5 ml of water and loaded onto 1 ml Co⁺² -Talon Metal Affinity column equilibrated with PBS. The column was exhaustively washed with PBS and CIBP-4 eluted with 2 ml of 1 M imidazole in PBS. To remove imidazole from AF647-CIBP-4 conjugate, the sample was concentrated to approximately 200 µl on Biomax (M.W. cut-off 5,000), diluted to original volume with PBS and concentrated again. That process of concentration/dilution was repeated three times. Final volume 0.5 ml (0.14 mg/ml). Recovery 86%.

### Confocal microscopy studies

HBEC (100,000 cell/well in a 24-well format plate) and U87MG (50,000) were respectively seeded on human fibronectin- (40 µg/ml) or poly-L-lysine-coated cover slips (Bellco Biotechnology) in 400 µl HBEC/U87MG media and grown until reached 80% confluence. Cells were then washed twice with DME and incubated in DME for 30 min at 37°C. Then, DME was removed and replaced with 250 µl/well of phenol red-free DME containing 100 nM AF647-CIBP-4 conjugate and 150 nM Lysotraker™ solution (Invitrogen) for 90 min. In another set of experiments, cells were incubated with 250 µl/well of phenol red-free DME containing 100 nM AF647-CIBP-4 conjugate for 75 min and then 2X dilution of Magic Red™ Cathepsin B detection solution (Immunochemistry Technologies) was added for additional 15 min. Cells were counterstained with the membrane dye DiOC5(3) for 15 seconds and then washed with PBS. Imaging of cells was performed using Zeiss LSM 410 (Carl Zeiss, Thornwood, NY, USA) inverted laser scanning microscope equipped with an Argon\Krypton ion laser and a Plan- Apochromat 63X, NA 1.4. Confocal images of two fluoroprobes were sequentially obtained using 488, 647 and 540-590 nm excitation laser lines to detect DiOC5(3) (510-525 nm emission) and Alexa 647 (670-810 nm emission) and Magic Red™ Cathepsin B (>610 nm) fluorescence.

### Capillary-like tube (CLT) formation and intracellular cathepsin B activity assays

In vitro angiogenesis was assessed by endothelial tube formation in growth factor reduced Matrigel™ (BD Bioscience, Bedford, MA). 24-well plates were coated with 300 µl of unpolymerized Matrigel™ (5-7 mg prot/ml) and allowed to polymerize for 90 min at 37°C. HBEC (40,000 cells) were suspended in 500 µl of either DME, serum-free U87MG CM (collected as described in Cell Cultures) or growth factors (VEGF, IGF-1, bFGF) in the absence or presence of 20 nM of either full length recombinant IGFBP-1 (IBP1, produced at BRI-NRC), IGFBP-2 (IBP2, produced at BRI-NRC and purchased from R&D systems, Minneapolis), IGFBP-3 (IBP3, from R&D systems), IGFBP-4 (IBP4, produced at BRI-NRC), IGFBP-5 (IBP5, produced at BRI-NRC and purchased from R&D systems) and IGFBP-6 (IBP6, from R&D systems) or the C-terminal protein fragments of IGFP-1 (CIBP1), IGFBP-2 (CIBP2), IGFBP-4 (CIBP4), IGFBP-5 (CIBP5) and IGFBP-6 (CIBP6), all produced at BRI-NRC). The ability of a synthetic membrane permeable cathepsin B inhibitor (CA074-ME, EMD Biosciences, Canada) to inhibit angiogenes and cathepsin B activity was also studied. CLT formation was analyzed after 24 h using an Olympus 1 X50 microscope. Phase contrast images were captured with a digital video camera (Olympus U-CMT) and analyzed using Northern Eclipse v.5.0 software. Experiments were repeated three times.. In order to determine the levels of cathepsin B activity in each experimental condition, at the end of the experiment, 6 µl of 26X Magic Red™ Cathepsin B reagent (Immunochemistry Technologies, LLC) were added to all the wells and maintained at 37°C in dark for two hours. Cells were washed twice with HBSS and intracellular fluorescence quantification (530/25 nm excitation and 645/40 nm emission) was performed using a cytofluorimeter plate reader (Bio-Tek FL600).

### U87MG growth in semi-solid agar

U87MG cell growth in semi-solid agar was determined in the absence or presence of 20 nM of either CIBP-4, CIBP-5 or dybutyril cAMP (dB-cAMP) as described previously (Moreno et al., 2006). Approximately 15,000 cells ± treatment were resuspended in 150 µl medium containing 0.3% agar, and seeded onto a well of a 24-well plate previously layered with 250 µl 0.6% agar. The solidified cell layer was covered with 50 µl DME ± treatment which was replaced every three days over a 21 day period. Phase contrast images (6 fields/well) were captured using a digital video camera (Olympus U-CMT) and analyzed with Northern Eclipse v.5.0 software. Color images were transformed to grey scale, thresholded, and then converted to binary images. Number and area of colonies per field were calculated. In order to measure cell viability, at the end of the experiment, 40 µl alamar blue was added to each well and fluorescence readings were performed every 10 min for a period of 180 min. Experiments were repeated 2-3 times in triplicates.

### Cathepsin B activity assay

U87MG cells were plated at a density of 10⁴ cells/well in 500 µl of U87MG media on poly-L-lysine-coated 24-well plates. Three days later, U87MG media was removed, cells rinsed twice with HBSS and incubated for 2 h or 18 h in 300 µl of either DME or DME supplemented with either 20 nM of the full length proteins IBP1, IBP2, IBP3, IBP4, IBP5, IBP6 or 20 nM the C-terminal CIBP1, CIBP2, CIBP4, CIBP5, CIBP6. Then, 6 µl of 26X Magic Red™ Cathepsin B reagent (Immunochemistry Technologies, LLC) were added to all the wells and maintained at 37°C in dark for two additional hours. Cells were then washed twice with HBSS before intracellular fluorescence quantification (530/25 nm excitation and 645/40 nm emission) in a cytofluorimeter plate reader (Bio-Tek FL600). Fluorescence readings for each treatment were normalized to their corresponding cellular protein content measured by Lowry method.

Levels of cathepsin B activity were also measured in U87MG CM alone or pre-incubated for 18 hours with 20 nM of IBP-1, IBP-2, IBP-3, IBP-4, IBP5, IBP-6, CIBP1, CIBP2, CIBP4, CIBP5, CIBP6 or 10 µg/ml CA074-Me (EMD Biosciences).

### Experimental glioma assay

Fertilized chicken (Gallus gallus) eggs were obtained from the Canadian Food Inspection Agency and placed into an egg incubator at 37 °C and 67-70% humidity (day 0). At day 3, windows were cut in the egg shell and covered with surgical tape (Durapore,) until day 10. Then, a sterile Nunc Thermanox (Nunc Inc., Naperville,. IL) plastic ring was placed onto the CAM, the delimited surface gently lacerated with a scalpel blade and a pellet of 10⁶ U87MG cells deposited into the center of the ring. At days 11-14, thirty µl of either sterile water + 6% DMSO alone (vehicle) or in combination of 250 nM of CIBP-4 was applied to the tumors. Digital photos were taken using a Canon 40D camera. At day 17, tumors were carefully removed from the CAM and weighted.

### Example 1: Human Brain Endothelial Cells (HBEC)

Confocal microscopy indicates that the C-terminal (CIBP-4, nt 155-258) protein fragment of IGFBP-4 conjugated to alexa fluor 647 (CIBP-4-AF147) internalizes into human brain endothelial cells (HBEC). The proteins show punctuate perinuclear localization in vesicle-like structures (Fig. 1). This indicates that CIBP-4 most likely recognizes and binds specific proteins contained in these vesicles.

Co-localization of CIBP-4 with lysosomes in HBEC using Lysotracker™ (a permeable acidotropic probe for selective fluorescent labeling of lysosomes) (Fig. 2) confirms that vesicles targeted by CIBP4 are lysosomes.

It has been disclosed in copending PCT application PCT/CA2006/000250 that CIBP-4 can inhibit angiogenic response induced by U87MG conditioned media and by different growth factors, including bFGF, VEGF and IGF-1. To determine the ability of these angiogenic factors to stimulate cathepsin B activity in HBEC, a membrane permeable cathepsin B target sequence peptide (Arginine-Arginine) linked to an amide substituted fluorophore, cresyl violet (Magic Red™ Immunochemistry Technologies, LLC) was used. Following enzyme cleavage at the arginine amide linkage site, the cresyl violet fluorophore generates red fluorescence when excited at 550-590 nm. It was found that U87MG CM, VEGF (20 ng/ml), IGF-1 (150 ng/ml) and bFGF (20 ng/ml) induce intracellular cathepsin B activity in HBEC seeded on Matrigel (Fig. 3 A-C, Fig. 4 A-D, Fig. 5 A-D). This indicates that angiogenesis induced by growth factors is associated with increased levels of intracellular cathepsin B activity in endothelial cells.

Since all IGFBP family members (1-6) have a thyroglobulin type I domain in their C-terminal sequence, and other unrelated proteins bearing the thyroglobulin type I domain have been shown to have anti-protease (mainly anti-cathepsin) activity, the ability of the C-terminal IGFBP-4/CIBP-4 (produced at BRI-NRC), to inhibit intracellular cathepsin B activity was analyzed. As shown in Fig. 3A-C, there is a strong correlation between the of intracellular cathepsin B activity and reduction in CLT formation exerted by CIBP-4 in HBEC cells seeded on Matrigel. 3D).

In order to determine the ability of other members of IGFBP family and their respective C-terminal fragments (containing the thyroglobulin type-1 domain) to inhibit angiogenesis (CLT formation by HBEC in Matrigel) and cathepsin B activity, the recombinant proteins IGFBP-1 (IBP1, Seq ID. 1), IGFBP-2 (IBP2, Seq ID. 3), IGFBP-5 (IBP5, Seq ID. 7) and the C-terminal fragments of IGFBP-1 (CIBP1, Seq ID. 2), IGFBP-5 (CIBP5, Seq ID. 8) and IGFBP-6 (CIBP6, Seq ID. 9) were produced at BRI-NRC using an optimized in house method. IGFBP-2 and IGFBP-5 from R&D systems were used as positive controls, to compare their efficacy to that of the corresponding BRI-NRC produced proteins. As shown in Figs 4-5, all the IGFBP members and their corresponding C-terminal fragment were potent inhibitors of both angiogenesis (CLT formation by HBEC in Matrigel) and cathepsin B activity, with the exception of IGFBP-2 that did not inhibit the angiogenic response and intracellular cathepsin B activity induced in HBEC by either U87MG CM or bFGF. However, IGFBP-2 was able to completely inhibit the angiogenic response and intracellular cathepsin B activity induced by VEGF and IGF-1.

These results also indicate that IGFBP family members, especially their C-terminal fragment that contains a thyroglobulin type-I domain, are potent inhibitors of angiogenesis most likely due to their capacity to inhibit cathepsin B activity in endothelial cells.

### Example 2: Human Glioblastoma Cells (U87MG)

To determine whether the anti-tumorigenic properties of CIBP4 (as disclosed in copending PCT application PCT/CA2006/000250) are associated with its ability to inhibit cathepsin B activity, confocal microscopy was performed to map intracellular cathepsin B activity in U87MG cells. As shown in Fig. 6, high levels of cathepsin B activity were observed in U87MG cells predominantly in the cytoplasm and the plasma membrane along side the cellular processes.

Co-localization of CIBP-4 conjugated to alexa fluor 647 with lysosomes was confirmed in U87MG cells using Lysotracker™ (Fig. 7)

Very high levels of secreted cathepsin B activity were also measured in U87MG CM and the activity was partially inhibited (20-70%) by overnight incubation with IGFBP family members and their C-terminal fragments (Fig. 8); the order of efficiency being CIBP2 (∼70%) > IBP2 = IBP6 = CIBP6 (∼60%) > CA074-ME (∼40%).

Intracellular evaluation of cathepsin B activity using Magic Red™ in U87MG also confirmed very high levels of activity in basal conditions. The intracellular cathepsin B activity was inhibited (∼50%) by overnight incubation of the cells with 20 nM of IBP2 (both from BRI-NRC and R&D systems), CIBP2 (produced by BRI-NRC), IBP5 (both from BRI-NRC and R&D systems) and CIBP5 (produced by BRI-NRC). These results indicate that IGFBP proteins can inhibit both intracellular and extracellular cathepsin B activity in glioblastoma tumor cells.

As disclosed in copending PCT application PCT/CA2006/000250, IGFBP-4 and the C-terminal IGFBP-4 fragment (CIBP-4) were able to reduce U87MG colony formation in soft agar. We now investigated the ability of C-terminal fragments of the other IGFBP members to inhibit U87MG colony formation in soft agar. As shown in Fig. 10, the efficacy of the fragments was: CIBP1 = CIBP6 (∼25%) < CIBP2 = CIBP4 (∼50%) < dB-cAMP (positive control, ∼ 65%) < CA074-ME (∼70%). This indicates, that cathepsin inhibition, as demonstrated with the synthetic cathepsin B inhibitor, blocks glioblastoma tumor growth and that the IGFBP members, specially CIBP2 and CIBP4 are potent inhibitors of both cathespin B activity and tumor growth.

The ability of CIBP4 (250 ng/ml) to block tumor growth was tested using the experimental glioma assay (Fig. 4A). The growth of U87MG cells on CAM was significantly (p<0.005) reduced (20-25%) in the CIBP4-treated compared to the vehicle-treated group. Analysis of tumor weight frequency distribution in three main groups (small size: 0-15 mg, medium size: 15-20 mg and large size: 20-36 mg) indicate that CIBP-4 treatment induces a shift (∼ 3-fold) towards smaller tumors [3-fold reduction in number of large size tumors (20-36 mg) versus a three-fold increase in small size tumors (0-15 mg)] compared to the vehicle-treatment (Fig. 3C). The average size of the tumors in each interval was similar between CIBP4-treated and vehicle treated group. These results indicate that CIBP4 inhibits tumor growth in an in vivo experimental glioma model.

Other advantages that are inherent to the structure are obvious to one skilled in the art. The embodiments are described herein illustratively and are not meant to limit the scope of the invention as claimed. Variations of the foregoing embodiments will be evident to a person of ordinary skill and are intended by the inventor to be encompassed by the following claims.

## Claims

1. Use of a peptide consisting of an amino acid sequence with at least 85% identity to amino acids 1-328 of SEQ ID No. 3 or amino acids 107-328 of SEQ ID No. 3 in the preparation of a medicament for inhibiting angiogenesis.

2. Use of a peptide consisting of an amino acid sequence with at least 85% identity to amino acids 1-328 of SEQ ID No. 3 or amino acids 107-328 of SEQ ID No. 3 in the preparation of a medicament for inhibiting cathepsin activity.

3. Use of a peptide consisting of amino acids 1-328 of SEQ ID No. 3 or amino acids 107-328 of SEQ ID No. 3 in the preparation of a medicament for inhibiting angiogenesis.

4. Use of a peptide consisting of amino acids 1-328 of SEQ ID No. 3 or amino acids 107-328 of SEQ ID No. 3 in the preparation of a medicament for inhibiting cathepsin activity.

5. Use of a peptide comprising an amino acid sequence with at least 85% identity to amino acids 1-328 of SEQ ID No. 3 or amino acids 107-328 of SEQ ID No. 3 in the preparation of a medicament for inhibiting cathepsin activity.

6. Use of a peptide comprising amino acids 1-328 of SEQ ID No. 3 or amino acids 107-328 of SEQ ID No. 3 in the preparation of a medicament for inhibiting cathepsin activity.

## Patentansprüche

1. Verwendung eines Peptids, das aus einer Aminosäuresequenz besteht, die mindestens zu 85% identisch zu den Aminosäuren 1-328 der SEQ ID Nr. 3 oder den Aminosäuren 107-328 der SEQ ID Nr. 3 ist, zur Herstellung eines Medikaments zur Hemmung der Angiogenese.

2. Verwendung eines Peptids, das aus einer Aminosäuresequenz besteht, die mindestens zu 85% identisch zu den Aminosäuren 1-328 der SEQ ID Nr. 3 oder den Aminosäuren 107-328 der SEQ ID Nr. 3 ist, zur Herstellung eines Medikaments zur Hemmung der Aktivität von Cathepsin.

3. Verwendung eines Peptids, das aus den Aminosäuren 1-328 der SEQ ID Nr. 3 oder den Aminosäuren 107-328 der SEQ ID Nr. 3 besteht, zur Herstellung eines Medikaments zur Hemmung der Angiogenese.

4. Verwendung eines Peptids, das aus den Aminosäuren 1-328 der SEQ ID Nr. 3 oder den Aminosäuren 107-328 der SEQ ID Nr. 3 besteht, zur Herstellung eines Medikaments zur Hemmung der Aktivität von Cathepsin.

5. Verwendung eines Peptids, das eine Aminosäuresequenz umfasst, die mindestens zu 85% identisch zu den Aminosäuren 1-328 der SEQ ID Nr. 3 oder den Aminosäuren 107-328 der SEQ ID Nr. 3 ist, zur Herstellung eines Medikaments zur Hemmung der Aktivität von Cathepsin.

6. Verwendung eines Peptids, das die Aminosäuren 1-328 der SEQ ID Nr. 3 oder die Aminosäuren 107-328 der SEQ ID Nr. 3 umfasst, zur Herstellung eines Medikaments zur Hemmung der Aktivität von Cathepsin.

## Revendications

1. Utilisation d'un peptide consistant en une séquence d'acides aminés avec au moins 85 % d'identité par rapport aux acides aminés 1-328 de la SEQ ID N°3 ou par rapport aux acides aminés 107-328 de la SEQ ID N°3 dans la préparation d'un médicament pour inhiber l'angiogénèse.

2. Utilisation d'un peptide consistant en une séquence d'acides aminés avec au moins 85 % d'identité par rapport aux acides aminés 1-328 de la SEQ ID N°3 ou par rapport aux acides aminés 107-328 de la SEQ ID N°3 dans la préparation d'un médicament pour inhiber l'activité de la cathépsine.

3. Utilisation d'un peptide consistant en les acides aminés 1-328 de la SEQ ID N°3 ou en les acides aminés 107-328 de la SEQ ID N°3 dans la préparation d'un médicament pour inhiber l'angiogénèse.

4. Utilisation d'un peptide consistant en les acides aminés 1-328 de la SEQ ID N°3 ou en les acides aminés 107-328 de la SEQ ID N°3 dans la préparation d'un médicament pour inhiber l'activité de la cathépsine.

5. Utilisation d'un peptide comprenant une séquence d'acides aminés avec au moins 85 % d'identité par rapport aux acides aminés 1-328 de la SEQ ID N°3 ou par rapport aux acides aminés 107-328 de la SEQ ID N°3 dans la préparation d'un médicament pour inhiber l'activité de la cathépsine.

6. Utilisation d'un peptide comprenant les acides aminés 1-328 de la SEQ ID N°3 ou les acides aminés 107-328 la SEQ ID N°3 dans la préparation d'un médicament pour inhiber l'activité de la cathépsine.
